# EUROPEAN PATENT SPECIFICATION

(11) **EP 0 661 069 B1**
(45) Date of publication and mention of the grant of the patent: **23.06.1999**
(21) Application number: 94309812.9
(22) Date of filing: 23.12.1994
(51) Int. Cl.: A61M 11/00, B05B 1/14, B05B 11/00, B05B 1/34

(54) **Spray container for spraying medicinal liquid to mucous membrane of throat**
Medizinischer Spraybehälter
Vaponisateur médical

(30) Priority: 28.12.1993 JP 7475593
(43) Date of publication of application: 05.07.1995
(73) Proprietor: Meiji Seika Kaisha, Ltd., Tokyo 104-8002 (JP)
(72) Inventor: Hattori, Masayuki, Pharmaceutical Research Center, Kohoku-ku, Yokohama-shi, Kanagawa (JP); Mastuo, Koichi, Pharmaceutical Research Center, Kohoku-ku, Yokohama-shi, Kanagawa (JP)
(74) Representative: Crouch, David John

(56) References cited:
- EP-A- 0 494 803
- EP-A- 0 504 509
- WO-A-93/23174
- FR-A- 2 403 115
- GB-A- 2 110 568
- PATENT ABSTRACTS OF JAPAN vol. 017 no. 206 (M-1400) ,22 April 1993 & JP-A-04 347360 (ISUZU MOTORS LTD) 2 December 1992,

## Description

This invention relates to an improvement to a spray container for spraying medicinal liquid to inflamed or otherwise troubled mucous membrane of the throat for treatment.

In WO 93/23174 there is disclosed a dispenser having a pushbutton mounted on a valve or a pump of the dispenser. This enables, by actuation of the pushbutton, liquid contained in a container to be sprayed through at least two spraying nozzles provided in the pushbutton and connected to a central channel of the pushbutton. Each nozzle has an axis oriented substantially transversally with respect to the axis of the central channel of the pushbutton. The axes of the spraying nozzles of the pushbutton are situated in the same plane, the axes of the spraying nozzles form between each other an angle between 1° and 40° and the nozzles are supplied by means of two conduits whose axes are parallel. However, such a dispenser is not suitable for medicinal sprays injected into the throat.

Spray containers are popularly used in recent years for spraying medicinal liquid to inflamed or otherwise troubled mucous membrane of the throat as well as for other purposes. Since conventional spray containers of the form under consideration comprises a nozzle tip provided with a single spray port through which medicinal liquid is injected in the form of a single cylindrical stream, they can cover only a small area of the membrane with a single injection action. If, for example, liquid is injected towards the mucous membrane of the throat from a spray container having a spray port with a diameter of 0.4mm and separated from the membrane by 10cm, only a surface area of 1.5cm² of the membrane is covered by liquid with a single injecting action so that liquid has to be injected for a number of times with varied angles of injection in order to fully cover the entire mucous membrane of the throat.

Medicinal liquid needs to be injected in the form of a cylindrical stream in order to prevent it from being inadvertently inhaled into the bronchi and then into the lungs. However, this arrangement is accompanied by the problem that the applied medicinal liquid is, at least partly, swallowed down into the digestive system as the amount of liquid increases with repeated injection actions.

In view of the above identified problem of conventional spray containers, it is therefore an aim of the invention to provide a spray container with a nozzle tip that can spray medicinal liquid to cover a remarkably large area of the mucous membrane of the throat in a single spraying action.

According to the invention, the above aim is addressed by providing a spray container comprising a spray head and a container main body wherein said spray head has a nozzle tip and said nozzle tip is provided with a pair of spray ports. Said spray ports may be horizontally juxtaposed.

With such an arrangement, a remarkably large area of the mucous membrane of the throat can be covered by medicinal liquid with a single spraying action so that the entire surface of the mucous membrane of the throat can be covered by liquid with a significantly reduced number of spraying actions as compared with the case using a conventional spray container and consequently the amount of medicinal liquid that is swallowed down by the subject can be significantly reduced.

Additionally, a desired profile of injected medicinal liquid and a desired coverage of medicinal liquid on the mucous membrane of the throat can be obtained by appropriately selecting the diameter of the spray ports and the distance separating the spray container from the mucous membrane.

Now, a preferred embodiment of the present invention will be described by way of example by referring to the accompanying drawings in which:
- Figure 1: is a partially sectional schematic side view of a preferred embodiment of the invention, showing only a principal portion thereof;
- Figure 2: is a partially sectional schematic front view of the embodiment of Figure 1;
- Figure 3: is an enlarged schematic front view of the nozzle tip of the embodiment of Figure 1;
- Figure 4: is a view similar to Figure 3, but showing the rear side of the nozzle tip; and
- Figure 5: is a partially sectional schematic plan view of a half portion of the nozzle tip of Figure 3.

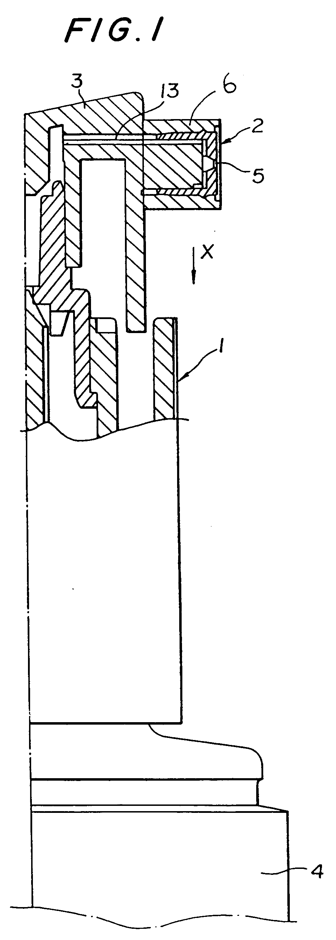

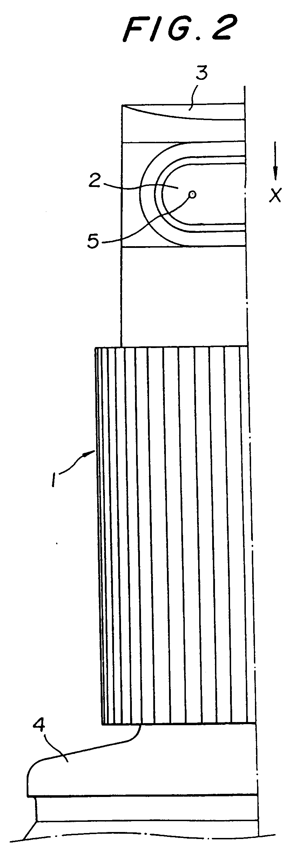

Figures 1 through 5 illustrate an embodiment of spray container for spraying a medicinal liquid to a mucous membrane of a throat in accordance with the present invention. The spray container is generally denoted by reference numeral 1 and comprises a spray head 3 provided with a nozzle tip 2 and a container main body 4 for storing a medicinal liquid. The spray container includes a pumping mechanism. When the spray head 3 provided with the nozzle tip 2 is depressed by a finger along the direction indicated by arrow X, the medicinal liquid in the container main body 4 is pumped up from the container main body 4 and injected through a pair of spray ports 5 and 8 of the nozzle tip 2 toward the mucous membrane of the throat. Since the hydrodynamics or the pumping mechanism of this spray container is the same as that of any known conventional spray containers, it will not be specifically described any further nor illustrated in the accompanying drawings.

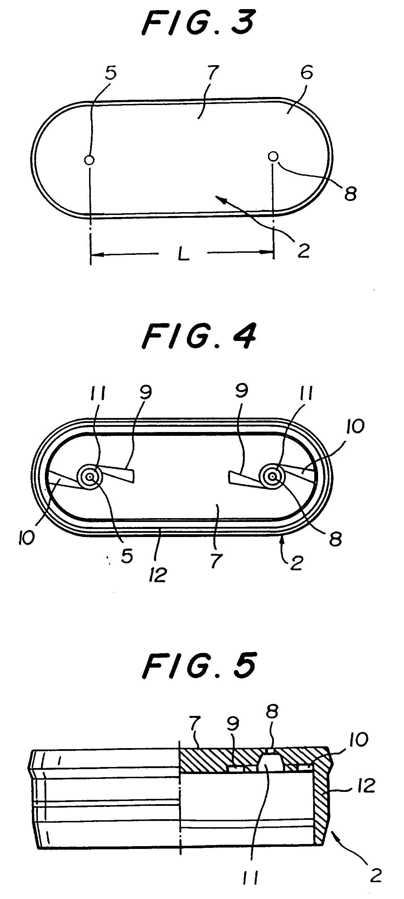

In the illustrated embodiment, the spray head 3 of the spray container 1 for spraying the medicinal liquid comprises a nozzle main body 6 and a nozzle tip 2. The nozzle tip comprises an elliptical front panel 7 and a peripheral wall 12. The elliptical front panel 7 is provided with a pair of spray ports 5, 8. The pair of spray ports 5, 8 are horizontally juxtaposed on the elliptical front panel 7 and separated from each other by a distance L as shown in Figures 3, 4 and 5.

As seen from Figures 4 and 5, the front panel 7 is provided at a rear side thereof with a pair of nozzle sacs 11, 11 coaxially aligned with the respective spray ports 5, 8. A pair of spin grooves 9, 10 are formed at an outer periphery of the nozzle sac 11, so that the medicinal liquid is injected from each of the spray ports 5, 8 as a spiral vortex.

In Figures 4 and 5, the peripheral wall 12 of the nozzle tip 2 is used for rigidly and engagedly fitting the nozzle tip 2 into a front end of a medicinal liquid flow path 13 of the spray head 3.

The spray container 1 may be made of synthetic resin.

In an experimental example of the present invention, the nozzle tip provided with a pair of spray ports 5 and 8 having a diameter of 0.25mm and being separated from each other by a distance L of 7mm was used with the above described conventional pumping mechanism and the container main body. On the other hand, a conventional nozzle tip having a single spray port with a diameter of 0.4mm was used. According to the experimental example of the present invention, an area covered by a single spraying action of the nozzle tip located 10cm away from a target was 2.2cm². On the other hand, according to the above described conventional nozzle tip, the area covered by a single spraying action of the nozzle tip located 10cm away from a target was 1.5cm². This means that a spray container made in accordance with the present invention can show an increase of approximately 46% in the area covered by a single spraying action and located 10cm away from the target.

The diameter of the injection port and the distance L are not limited to the above described experimental example.

While a nozzle tip in accordance with the invention can remarkably increase the coverage of sprayed medicinal liquid by single spraying action, the amount of medicinal liquid consumed in the spraying action is substantially the same as the amount consumed by a comparable conventional nozzle tip, because the diameter of the injection ports can be reduced to about 60% of that of a conventional injection port.

In accordance with the present invention, a remarkably large area of the mucous membrane of the throat can be covered by medicinal liquid with a single spraying action by providing a pair of spray ports on the nozzle tip, so that the entire surface of the mucous membrane of the throat can be covered by liquid with a significantly reduced number of spraying actions as compared with the case of using a conventional spray container and consequently the amount of medicinal liquid that is swallowed down by the subject can be significantly reduced.

Additionally, a desired profile of injected medicinal liquid and a desired coverage of medicinal liquid on the mucous membrane of the throat can be obtained by appropriately selecting the diameter of the spray ports and the distance between the spray ports.

## Claims

1. A spray container (1) for spraying a medicinal liquid to a mucous membrane of a throat, comprising
a spray head (3), a pumping mechanism and a container main body (4),
the spray head (3) having a nozzle tip (2), and
the nozzle tip (2) being provided with a pair of spray ports (5, 8) each having a construction which is such as to generate a generally cylindrical stream when in use.

2. A spray container (1) according to claim 1,
**characterised in that**
the nozzle tip (2) comprises a front panel (7) and a peripheral wall (12), and
the front panel (7) is provided with the spray ports (5, 8).

3. A spray container (1) according to claim 2,
**characterised in that**
a rear surface of the front panel (7) is provided with nozzle sacs (11), and
spin grooves (9, 10) are formed at an outer periphery of each of the nozzle sacs (11) so that medicinal liquid is injected from each of the spray ports (5, 8) as a spiral vortex.

4. A spray container (1) according to any preceding claim,
**characterised in that**
the spray ports (5, 8) are horizontally juxtaposed.

5. A spray container (1) according to any preceding claim,
**characterised in that**
the spray container (1) comprises a plastics resin.

6. A spray container (1) according to any preceding claim,
**characterised in that**
the diameter of each of the spray ports (5, 8) is substantially 0.25mm.

## Patentansprüche

1. Spraybehälter (1) zum Sprühen einer medizinischen Flüssigkeit auf eine Halsschleimhaut, mit
einem Spraykopf (3), einem Pumpmechanismus und einem Behälterhauptkörper (4),
wobei der Spraykopf (3) eine Düsenspitze (2) aufweist, und
die Düsenspitze (2) mit einem Paar Sprayöffnungen (5, 8) versehen ist, von denen jede eine derartige Ausbildung aufweist, daß bei Benutzung ein im wesentlichen zylindrischer Strom erzeugt wird.

2. Spraybehälter (1) nach Anspruch 1, **dadurch gekennzeichnet**, daß die Düsenspitze (2) eine Vorderwand (7) und eine Umfangswand (12) umfasst, und
die Vorderwand (7) mit den Sprayöffnungen (5, 8) versehen ist.

3. Spraybehälter (1) nach Anspruch 2, **dadurch gekennzeichnet**, daß die Rückseite der Vorderwand (7) mit Düsenkammern (11) versehen ist, und
Drallnuten (9, 10) auf einem Außenumfang jeder Düsenkammer (11) gebildet sind, so daß die medizinische Flüssigkeit von jeder Sprayöffnung (5, 8) als spiralenförmiger Wirbel abgesprüht wird.

4. Spraybehälter (1) nach einem der vorherigen Ansprüche, **dadurch gekennzeichnet**, daß die Sprayöffnungen (5, 8) horizontal nebeneinander angeordnet sind.

5. Spraybehälter (1) nach einem der vorherigen Ansprüche, **dadurch gekennzeichnet**, daß der Spraybehälter (1) aus einem Kunstharz besteht.

6. Spraybehälter (1) nach einem der vorherigen Ansprüche, **dadurch gekennzeichnet**, daß der Durchmesser jeder der Sprayöffnungen (5, 8) im wesentlichen 0,25 mm beträgt.

## Revendications

1. Pulvérisateur (1) pour projeter un liquide médicamenteux sur la membrane muqueuse de la gorge, comprenant
une tête de pulvérisation (3), un mécanisme de pompage et un corps principal formant récipient (4),
la tête de pulvérisation (3) présentant un embout de diffuseur (2), et
l'embout de diffuseur (2) étant pourvu d'une paire d'orifices de projection (5, 8) ayant chacun une structure permettant de produire, lors de l'utilisation, un flux globalement cylindrique.

2. Pulvérisateur (1) selon la revendication 1,
**caractérisé en ce que**
l'embout de diffuseur (2) comprend une plaque frontale (7) et une paroi périphérique (12), et
en ce que la plaque frontale (7) est pourvue des orifices de projection (5, 8).

3. Pulvérisateur (1) selon la revendication 2,
**caractérisé en ce que**
une surface arrière de la plaque frontale (7) est pourvue de poches de buse (11), et
en ce que des rainures en hélice (9, 10) sont formées au niveau d'une périphérie extérieure de chacune des poches de buse (11) de telle manière que le liquide médicamenteux soit injecté à partir des orifices de projection (5, 8), sous la forme d'un tourbillon en spirale.

4. Pulvérisateur (1) selon l'une quelconque des revendications précédentes,
**caractérisé en ce que**
les orifices de projection (5, 8) sont juxtaposés horizontalement.

5. Pulvérisateur (1) selon l'une quelconque des revendications précédentes,
**caractérisé en ce que**
le pulvérisateur (1) comprend une résine plastique.

6. Pulvérisateur (1) selon l'une quelconque des revendications précédentes,
**caractérisé en ce que**
le diamètre de chacun des orifices de projection (5, 8) est sensiblement de 0,25 mm.
